# EUROPEAN PATENT APPLICATION

(11) **EP 2 363 075 A1**
(43) Date of publication of application: **07.09.2011**
(21) Application number: 11153192.7
(22) Date of filing: 03.02.2011
(51) Int. Cl.: A61B 17/00

(54) **Device for preventing clot migration from left atrial appendage**

(30) Priority: 19.01.2011 US 8990; 12.02.2010 US 337972 P
(71) Applicant: Rex Medical, L.P., Conshohocken, PA 19428 (US)
(72) Inventor: McGuckin, Jr., James F, Radnor, PA 19087 (US); Bressler, James Erich, Langhorne, PA 19047 (US)
(74) Representative: Jackson, Derek Charles

(57) **Abstract**

A device for placement within the left atrial appendage (B) of a patient comprising a retention member (12, 112, 150, 212) and a material (30, 120, 160, 215) positioned within the retention member and unattached thereto. The retention member has a first elongated configuration for delivery and a second expanded configuration for placement within the left atrial appendage. The material is configured to float within the retention member. The retention member can have at least one appendage wall engagement member (14, 14', 114, 214) to secure the retention member to the appendage.

## Description

### Background Art

This application claims priority from provisional application serial no. 61/337,972, filed February 12, 2010, and is a continuation in part of application serial no. 12/151,790, filed May 9, 2008, which claims priority from provisional application serial no. 60/932,448, filed May 31, 2007, and is a continuation in part of application serial no. 11/978,821, filed October 30, 2007, which is a continuation of application serial no. 10/889,429, filed July 12, 2004, which claims priority from provisional application serial no. 60/572,274, filed May 18, 2004 and is a continuation in part of application serial no. 10/805,796, filed March 22, 2004, which claims priority from provisional application serial no. 60/538,379, filed January 22, 2004. The entire contents of each of these applications are incorporated herein by reference.

### Technical Field

This application relates to a device for preventing clot migration from the left atrial appendage of the heart.

### Background of Related Art

The atrial appendage is a small muscular pouch or cavity attached to the atrium of the heart. The left atrial appendage (LAA) is connected to the wall of the left atrium between the mitral valve and the left pulmonary vein. In proper functioning, the left atrial appendage contracts with the rest of the left atrium during a heart cycle, ensuring regular flow of blood.

Atrial fibrillation is the irregular and randomized contraction of the atrium working independently of the ventricles. This resulting rapid and chaotic heartbeat produces irregular and turbulent blood flow in the vascular system, resulting in the left atrial appendage not contracting regularly with the left atrium. Consequently, the blood can become stagnant and pool in the appendage, resulting in blood clot formation in the appendage. If the blood clot enters the left ventricle it can enter the cerebral vascular system and cause embolic stroke, resulting in disability and even death.

One approach to treatment is the administration of medications to break up the blood clots. However, these blood thinning medications are expensive, increase the risk of bleeding and could have adverse side effects. Another approach is to perform invasive surgery to close off the appendage to contain the blood clot within the appendage. Such invasive open heart surgery is time consuming, traumatic to the patient, increases patient risk and recovery time, and increases costs as extended hospital stays are required.

It is therefore recognized that a minimally invasive approach to closing off the appendage to prevent the migration of blood clots into the ventricle and cranial circulation would be beneficial. These devices, however, need to meet several criteria.

Such minimally invasive devices need to be collapsible to a small enough dimension to enable delivery through a small incision while being expandable to a sufficiently large dimension with sufficient stability to ensure sealing of the appendage is maintained. These devices also need to be atraumatic. Further, the size of the appendage can vary among patients and therefore the devices need to be expandable to the appropriate size to close off the appendage.

There have been several attempts in the prior art to provide minimally invasive appendage closure devices. For example, in U.S. Patent No. 6,488,689, a capture loop or clip is placed around the appendage to hold the appendage closed. These devices can be traumatic to the vascular structure. The Amplatzer occluder marketed by AGA Medical, provides for stent like expansion within a balloon. However, the diameter of expansion is not controllable and the collapsed configuration is relatively large, disadvantageously increasing the profile for insertion. In U.S. Patent No. 6,152,144, an occluding member having an outer rim and a thin mesh barrier to provide a seal is placed at the opening of the appendage. Radially extending shape memory members extend from the shaft to anchor the device. An expandable anchoring member is also disclosed. In another embodiment, an occlusive coil having a random configuration is placed in the appendage to induce clot. U.S. Patent Nos. 6,551,303 and 6,652,555 disclose a membrane placed across the ostium of the atrial appendage to prevent blood from entering. Various mechanisms such as shape memory prongs, anchors, springs and struts function to retain the membrane. These devices, however, suffer from various deficiencies.

Therefore, there is a need for an improved device for the left atrial appendage which will effectively block blood clot migration from the appendage, remain securely retained within the appendage, and have a reduced delivery profile to minimize the surgical incision and facilitate passage through the vascular system to the appendage.

### Summary of invention

The present invention overcomes the problems and deficiencies of the prior art. The present invention provides a device for placement in the left atrial appendage of a patient comprising a retention member and a material positioned within the retention member and unattached thereto. The retention member has a first elongated configuration for delivery and a second expanded configuration for placement within the left atrial appendage. The material is configured to float within the retention member in the expanded configuration of the retention member and cause blot clot within the appendage. The retention member has at least one appendage wall engagement member to secure the retention member to the appendage.

In some embodiments, in the second configuration, the retention member moves toward a shape memory position.

In one embodiment, the material comprises a mesh. In another embodiment, the material comprises a plurality of fibers. In another embodiment, the material comprises a plurality of ribbons. Combinations of these materials or use of other materials is also contemplated.

The present invention also provides in another aspect a device for placement in the left atrial appendage comprising a tube laser cut to form a series of struts, the tube having a first elongated configuration for delivery and a second configuration for placement. In the second configuration, the tube has an expanded configuration and the struts extend outwardly so that a distal region of the struts has a greater dimension than a proximal region and the struts define a space therebetween. A material is positioned within a region defined by the struts and unattached thereto for floating movement in the space between the struts, the material causing blood clots within the appendage.

In one embodiment, the material comprises a mesh. In another embodiment, the material comprises a plurality of fibers. In another embodiment, the material comprises a plurality of ribbons. Combinations of these materials or use of other materials is also contemplated.

In another aspect, a method for blocking blot clot migration from a left atrial appendage is also provided comprising the steps of inserting into the left atrial appendage a sheath containing a retention member having a plurality of struts in a reduced profile position, exposing the retention member from the sheath to enable it to expand to engage a wall of the left atrial appendage, subsequently inserting a material in situ within a space between the plurality of struts to enable the material to float within the space, and withdrawing the sheath to leave the retention member in the left atrial appendage so the material floats within the space defined by the plurality of struts to cause blood clots in the appendage.

Preferably, the retention member has a plurality of shape memory struts and the step of exposing the retention member enables the struts to move toward a shape memorized position.

### Brief description of drawings

Preferred embodiment(s) of the present disclosure are described herein with reference to the drawings wherein:

Figure 1 is a perspective view of one embodiment of a retention member of the left atrial appendage device of the present invention shown in the collapsed position for delivery;

Figure 2 is a transverse cross-sectional view taken along line 2-2 of Figure 1;

Figure 3 is a cross-sectional view taking along line 3-3 of Figure 1 showing a portion of the retention member within a delivery catheter;

Figure 4 is a perspective view showing the retention member in the expanded position with floating mesh material positioned therein;

Figure 4A is a perspective view showing an alternate embodiment of the retention member in the expanded position with floating mesh material positioned therein;

Figure 5 is a perspective view of an alternate embodiment of the left atrial appendage device of the present invention showing the retention member in the expanded position with floating fibers positioned therein;

Figure 6 is a perspective view of another alternate embodiment of the left atrial appendage device showing the retention member in the expanded position with floating ribbons positioned therein;

Figure 7 is an anatomical view showing insertion of the device of Figs. 1-4 through the femoral vein of a patient to access the left atrial appendage;

Figures 8-8D illustrate the steps of placement of the device of Figures 1-4 in the left atrial appendage wherein:

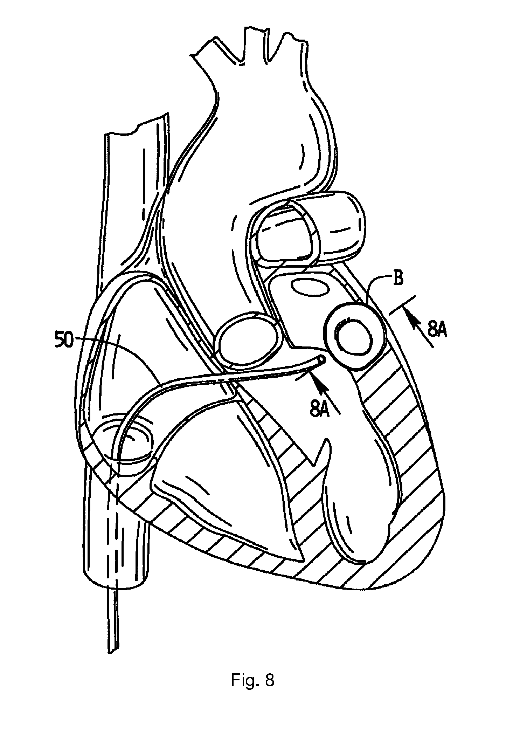

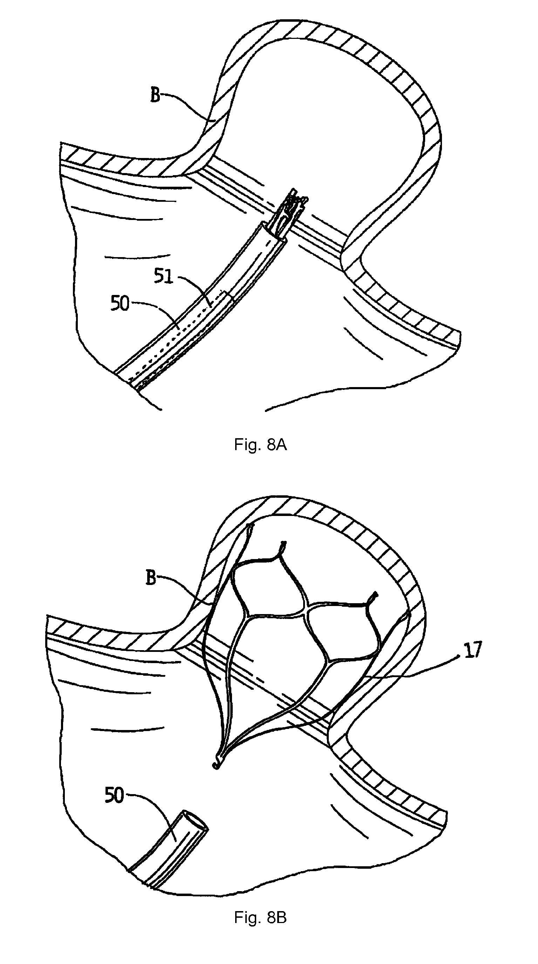

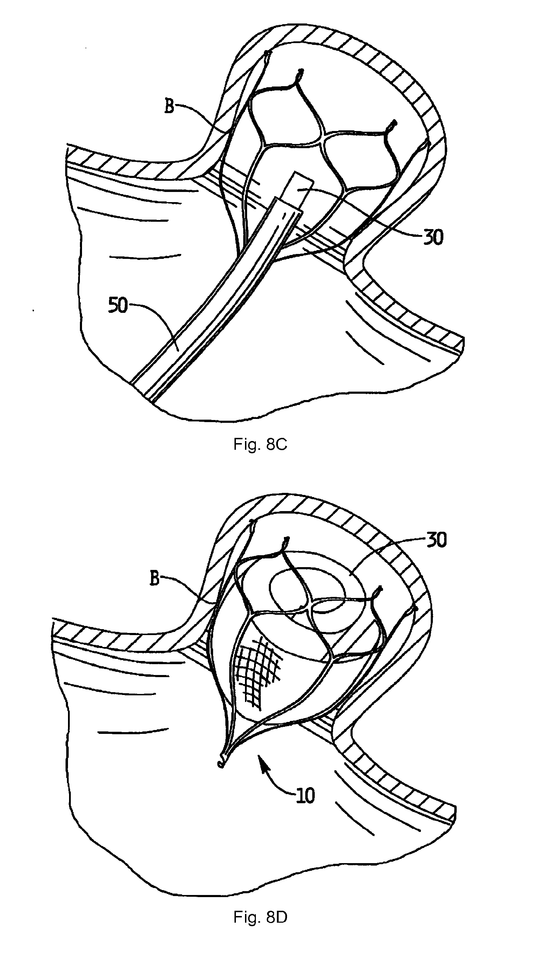

Figure 8 illustrates placement of the delivery catheter adjacent the left atrial appendage;

Figure 8A is a close up view illustrating initial deployment of the retention member of the left atrial appendage device;

Figure 8B is a close up view illustrating full deployment of the retention member;

Figure 8C is a close up view illustrating advancement of the delivery device into the retention member; and

Figure 8D illustrates the floating mesh inserted within the retention member;

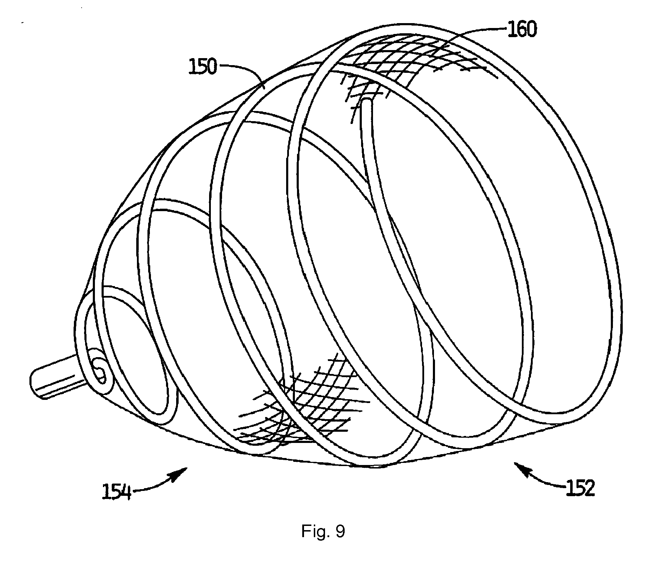

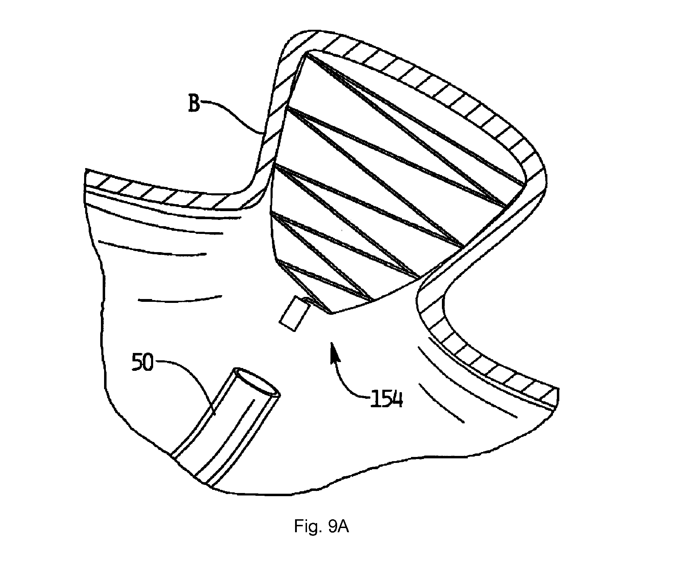

Figure 9 is a perspective view showing an alternate embodiment of the left atrial appendage device of the present invention showing the retention member in the expanded position with the floating mesh material positioned therein; and

Figure 9A illustrates placement of the device of Figure 9 in the left atrial appendage.

### Description of embodiments

Referring now in detail to the drawings where like reference numerals identify similar or like components throughout the several views, the present invention provides a device for blocking blood clot migration from the left atrial appendage ("LAA"). The device can be inserted minimally invasively. The device includes a retention (securement) member and material unattached to the retention member and movably positioned therein to cause blood clots after a period of time. The retention member provides for attachment to the appendage wall as well as a retention structure to retain within the appendage the various embodiments of the blood clotting material described below.

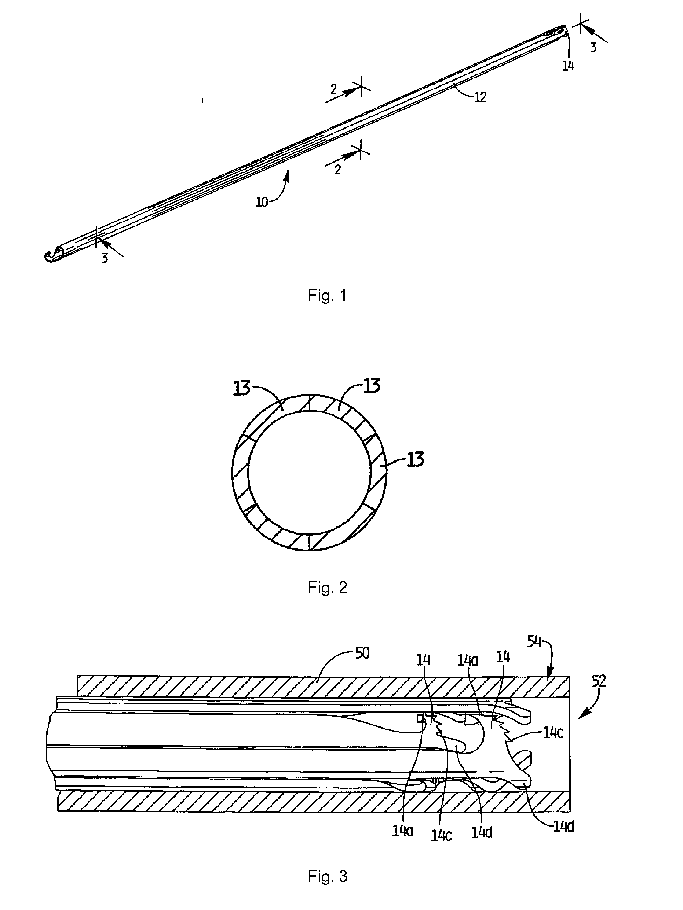

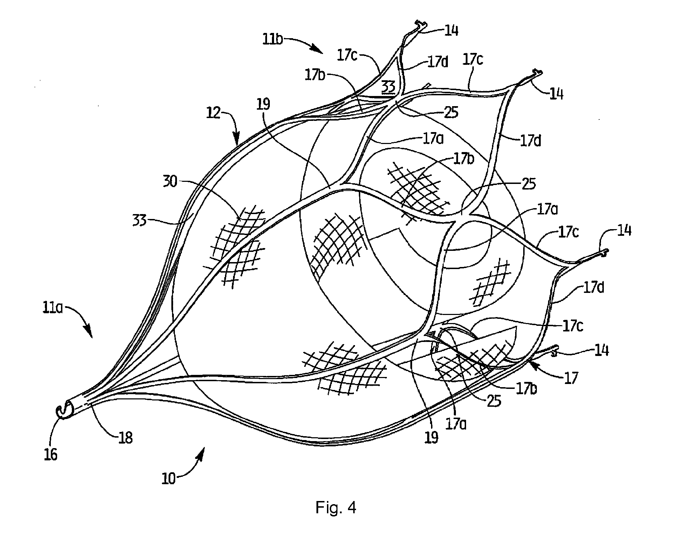

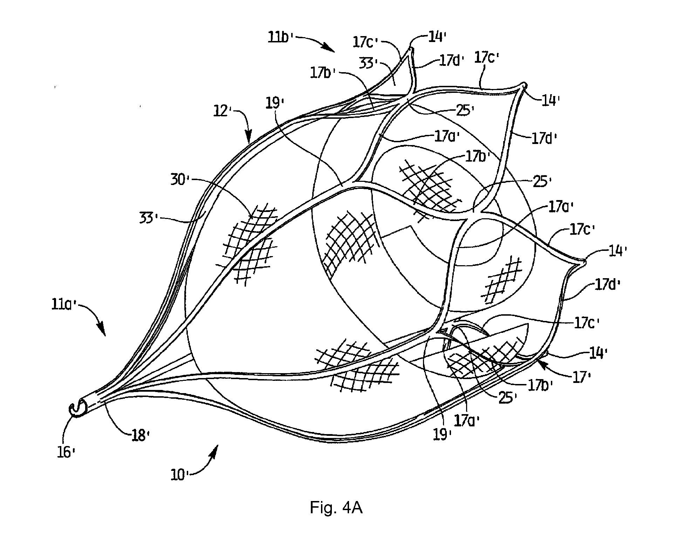

With initial reference to Figures 1-4 which show the left atrial appendage device 10 in the low profile delivery (collapsed) configuration for insertion (Figs. 1-3) and Fig. 4 which shows the device in the expanded configuration for placement, the device 10 includes a securement or retention component (member) 12. The retention member 12 forms a containment member to receive therein the material 30 for inducing blood clots. In the embodiment of Figures 1-4, the material comprises a mesh 30. The retention member 12 has engagement hooks 14 for engaging the appendage wall to retain the retention member 12 within the appendage. The mesh 30 is preferably advanced into the member 12 in situ as described below. Alternatively, the mesh can be positioned within the retention member 12 in the delivery position and then advanced together with the retention member 12 through the LAA opening. In the embodiment of Figure 1, floating within the retention member, and preferably free floating therein, is mesh 30, preferably made of a thrombogenic material, which causes blood clots and with the retention member 12 prevents migration of blood clots from the appendage. The device 10 is preferably formed from a laser cut tube, although other ways of forming the device are also contemplated.

The mesh is not shown in Figures 1-3 as in this embodiment the mesh is position proximal of the retention member 12 since it is delivered after the retention member 12 is placed in the body. The mesh could alternatively be delivered by a separate catheter after the delivery catheter 50 for the retention member 12 delivers the retention member 12 and is withdrawn. As can be appreciated, the configuration and dimension of the retention member 12 keeps the mesh 30 within the appendage while also providing enough space for movement of the material therein.

Turning to Figure 4 which illustrates the device 10 in the expanded (deployed) position, the retention member (component) 12 is in the form of a bell shaped device with struts as described in detail with respect to the filter disclosed in U.S. Patent No. 7,338,512, the entire contents of which are incorporated herein by reference. The device can alternatively have a retention (member component) in the form of the filter disclosed in U.S. Patent No. 7,704,266, the entire contents of which are incorporated herein by reference. The device 10, as shown in Figure 4A has a proximal end 11 a and a distal end 11 b. The retention member 12 is preferably composed of shape memory material, such as Nitinol, with an austenitic shape memorized position illustrated in Figure 4 and has a plurality of struts 17 emerging from apex 18 at proximal end 11a and terminating in wall engaging or retention hooks 14 at distal end 11 b. In this embodiment, six struts are provided although a different number of struts is also contemplated. A retrieval hook 16 is positioned on the proximal end 11a to enable the device 10 to be grasped by a snare or other device and removed if desired.

The struts 17 can be interconnected by interconnecting struts 17a, 17b that curve outwardly away from the central axis then inwardly toward each other to form a V-shaped end portion with hook 14. The connecting struts 17a, 17b are joined to connecting struts of adjacent struts at region 25 at a distal portion. Thus, a closed geometric shape 33 is formed which can be substantially oval, substantially diamond shaped, or other shapes. A fewer or greater number of closed shapes can be formed. That is, the struts 17 preferably divide at region 19 into two connecting struts 17a, 17b, angling away from each other, and then join at region 25, extending distally, then angle away from each other at struts 17c, 17d to join an adjacent interconnecting strut (17c or 17d) terminating in hooks 14. Thus, in one embodiment, the thickness of the connecting strut 17a, 17b is about half the thickness of the strut 17 proximal of the bifurcation and about half the thickness of the region 25. The interconnecting struts 17 help to provide a retention structure to restrain the floating material positioned inside component 12. Thus, the configuration and spacing of the struts 17 prevent the mesh (or other material) from migrating out of the appendage, while enabling free floating movement within the appendage. The interconnecting struts 17 also stiffen the device to enhance retention and increase the radial force. They also provide a more symmetric and uniform deployment. The hooks 14 are configured to engage the appendage wall for maintaining the position of the device 10. The struts are preferably flared and create a distal opening and a space between the struts. For clarity, not all the identical parts are labeled throughout the drawings. It should be appreciated that materials other than Nitinol or shape memory are also contemplated.

The hooks 14 preferably extend substantially perpendicular from the strut and can be formed by torquing the struts so the hooks bend out of the plane. Preferably, a first set of hooks is larger than a second set of hooks, although hooks of the same size are also contemplated. Preferably, when formed in a laser cut tube, the larger hooks are formed so that they occupy a region equivalent to the transverse dimension of two adjacent struts. Preferably, three smaller hooks and three larger hooks are provided in alternating arrangement in the embodiment utilizing six struts. The smaller hooks are preferably spaced axially with respect to each other and axially inwardly with respect to the larger hooks as in the filter hooks of U.S. Patent No. 7,704,266 to minimize the collapsed profile (transverse dimension) of the filter when collapsed for insertion. The penetrating tips 14a (Fig. 3) penetrate the tissue to retain the device 10, and preferably point toward the proximal end 11a of the device.

Each of the hooks 14 can have a series of teeth 14c to engage the appendage wall to provide additional retention to prevent movement of the device 10. A heel 14d can be provided which extends past the hook 14 to function as a stop to prevent the device from going through the wall. The angle of the heel 14d in the smaller hooks is preferably less than the angle in the larger hooks to provide room for nesting of the hooks as shown in Figure 3. For clarity, not all of the hooks are fully labeled.

In an alternate embodiment, the struts 17' terminate in blunt tips with the radial force of the struts maintaining the position of the device. This is shown for example in Figure 4A, wherein except for blunt tip 14' instead of hooks 14, device 10' is identical to device 10 of Figure 4, and identical parts are labeled with "prime" designations. For brevity, parts identical to those of Figure 4 are not further described as they are identical in structure and function to Figure 4 and thus the description relating to Figure 4 is fully applicable to the device of Figure 4A, except for the blunt tips 14' instead of hooks 14.

The retention (securement) member 12 is maintained in a substantially straightened softer martensitic configuration within the delivery catheter or sheath 50 for delivery as shown in Figure 3. The smaller hooks preferably nest within the larger hooks. Cold saline can be injected during delivery to maintain the struts 17 in this martensitic condition to facilitate exit from the distal opening 52 at the distal end portion 54 of catheter 50. When the struts 17 exit the delivery sheath (tube) 50, they are warmed by body temperature and move toward their illustrated memorized position as shown in Figure 4. Alternatively, they can be configured so that release from the sheath reduces the stress to enable the retention member 12 to return to its expanded memorized position.

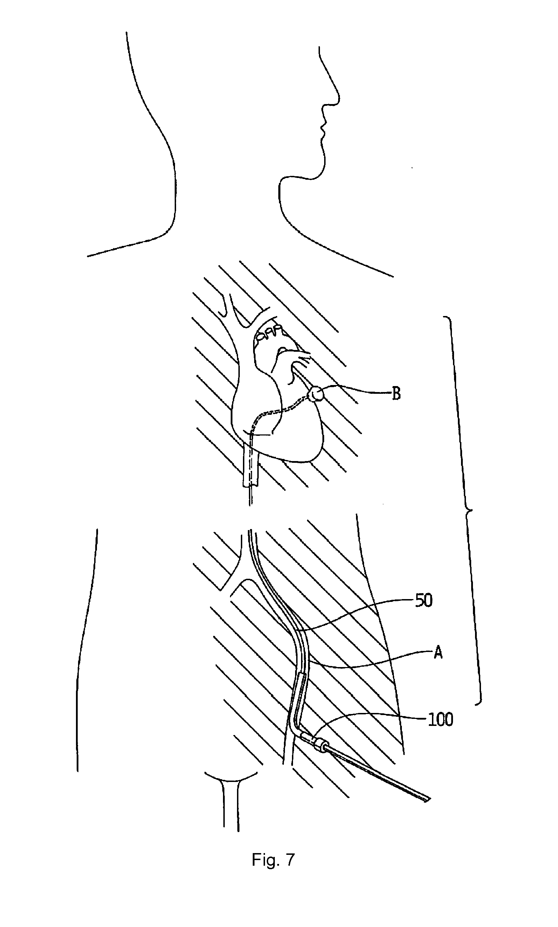

As shown in Figure 7, the device 10 is preferably inserted within delivery catheter 50 through the femoral vein A and advanced through the septum to access the left atrial appendage B. It is positioned in this embodiment with the distal end 11b further from (distal of) the appendage opening and the retrieval hook 16 proximal to the appendage opening, as shown in Figure 8B. When positioned in the appendage, the hooks 14 engage the wall to retain the device 10 in the appendage.

The device 10 in the embodiment of Figures 1-4 (and 4A) has mesh material unattached to and floating within the retention member 12. Preferably, the mesh material 30 is free-floating within the retention member 12. The amount of mesh material 30 is substantial enough to occupy a substantial space within the retention member 12 while still small enough to allow it to freely move within the space defined by the struts 17 of the retention member 12. It is also preferably of sufficient size to be retained by the struts 17. However, the mesh (and other material described herein) could also in some embodiments protrude through some of the struts, while still being retained in the appendage. The mesh 30 is preferably in the form a tightly woven material to provide sufficiently small spaces to effectively block blood large clot migration from the appendage while initially allowing blood flow therethrough. The material 30 is preferably of sufficient size to occupy a large percentage of the volume of the left atrial appendage. The mesh 30 functions to cause blood clotting. That is, once placed, blood flow continues through the device 10 until the mesh causes blood clotting, and eventually the clots can fill the volume, and in some applications the entire volume, of the left atrial appendage, with the large clots preventing migration.

The mesh 30 can be delivered within the retention member 12 such that in the collapsed position of the retention member 12, the mesh 30 is contained and compressed therein. After delivery, it would expand within the space of the retention member 12, i.e. within the space between the struts 17, since the struts expand when exposed from the delivery catheter.

In an alternate embodiment, the retention member 12 would be placed within the appendage first, and then once in place, the mesh 30 would be delivered through the spaces between the struts 17 for placement within the retention member 12.

The mesh 30 can be rolled up or folded for delivery. It can be one uniform piece or composed of two or more pieces of mesh.

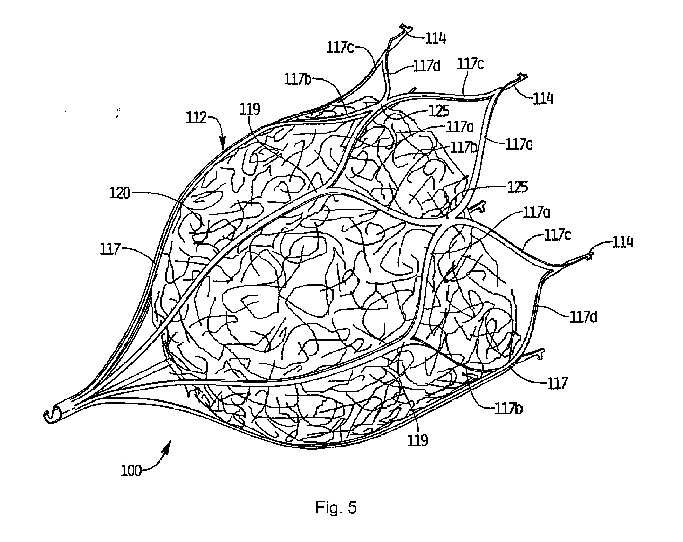

In an alternate embodiment, instead of the mesh floating within the space between the struts, the material to induce blood clotting can be in the form of unorganized fibers as shown in Figure 5. The fibers 120 can comprise a large number of threads, formed as separate pieces, and tangled or intertwined together. The fibers 120 can be compressed for delivery and then enlarge when released from the delivery catheter. The fibers 120 can be delivered inside the retention member 112 or alternatively subsequently placed between the struts of the device 100. That is, as with the mesh of the embodiment of Figure 4, the retention (securement) member 112 can be delivered with the fibers 120 positioned collapsed (compressed) therein, or alternatively, and preferably, the retention member 112 would be placed in the LAA first, followed by insertion of the fibers 120 in the spaces between the struts 117 of retention member 112 as described herein with respect to mesh 30.

The fibers 120, like the aforedescribed mesh, are unattached to the retention member 112 and are floating, and preferably free floating, within the space defined by the struts 117 of the retention member 112, causing blood clots in the same manner as described above with respect to the floating mesh of Figure 4 as they effectively block large blood flow clot migration from the appendage while initially allowing blood flow therethrough. The material (fibers) is preferably of sufficient size to occupy a large percentage of the volume of the left atrial appendage, and in some embodiments can fill the entire volume. The fibers function to cause blood clotting. That is, once placed, blood flow continues through the device 10 until the material 120 causes blood clotting. The retention (securement) member 112 is otherwise identical in structure and function to retention member 12 of Figure 1, and for convenience, identical parts are labeled in the "100" series, e.g. struts 117 bifurcate at region 119 into interconnecting struts 117a, 117b, join at region 125, then curve outwardly at interconnecting struts 117c, 117d to join another connecting strut and terminate in vessel engaging hooks 114, or alternatively, blunt ends. Consequently, these identical parts of retention member 112 for brevity are not described in further detail as the discussion of retention member 12 is fully applicable to retention member 112. The retention member 112 can alternatively be in the form of the filters of the 7,338, 512 and 7,704,226 patents incorporated by reference herein in their entirety.

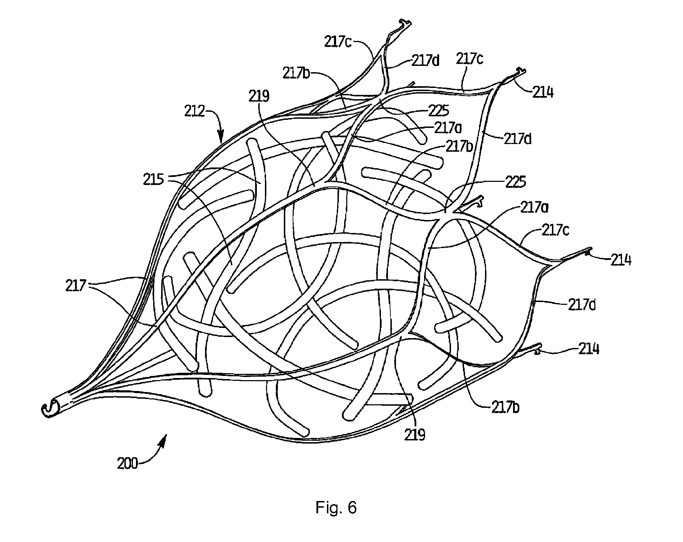

In an alternate embodiment of Figure 6, instead of the mesh floating within the space between the struts 217, the clotting material can be in the form of a plurality of ribbons 215 organized in a set pattern or alternatively randomly intertwined. The ribbons 215 are tangled or intertwined together. The ribbons 215 can be compressed for delivery and then enlarge with the struts when released from the delivery tube or alternatively placed between the struts 217 of the device 200 after the struts 212 are released and placed within the appendage. That is, as with the mesh of the embodiment of Figure 4, the retention (securement) member 212 can be delivered with the ribbons 217 positioned collapsed (compressed) therein, or alternatively, and preferably, the retention member 212 would be placed in the LAA first, followed by insertion of the ribbons 215 in the spaces between the struts 217 of retention member 212.

The ribbons 215, like the aforedescribed mesh, are unattached to the retention member 212 and float within the space defined by the struts 217 of the retention member 212, and, preferably free float, causing blood clots in the same manner as described above with respect to the floating mesh of Figure 4 as they effectively block large blood clot migration from the appendage while initially allowing blood flow therethrough. The material (ribbons) is preferably of sufficient size to occupy a large percentage of the volume of the left atrial appendage, and in some instances the entire volume. As with the mesh and fibers described herein, can be fully contained within the retention member 212 or extend beyond the struts. The ribbons function to cause blood clotting. That is, once placed, blood flow continues through the device 10 until the material causes blood clotting. The retention member 212 is otherwise identical to retention member 12 of Figure 1, and for convenience, identical parts are labeled in the "200" series, e.g. struts 217 divide at region 219 into interconnecting struts 217a, 217b, join at region 225, the extend outwardly at 217c, 217d, and terminate in vessel engaging hooks 214, or alternately, blunt ends. Consequently, these identical parts of retention member 212 for brevity are not described in further detail as the discussion of retention member 12 is fully applicable to retention member 212. The retention member 212 can alternatively be in the form of the filters of the 7,338,512 and 7, 704,266 patents previously incorporated herein by reference herein in their entirety.

The mesh (or other clot material such as ribbons or fibers) can be inserted with the retention member in a collapsed (compressed) state within the collapsed retention member or alternatively, if desired, can be delivered in situ within the opening between the struts in an already placed retention member. Such subsequent delivery could reduce the transverse dimension of the device in the collapsed position for delivery. The clot material can be inserted with the same catheter as the delivery catheter for the retention member or inserted by another catheter.

The method of placement of the device of the present invention will now be described for closing a left atrial appendage in conjunction with the embodiment of Figure 1 by way of example, with the mesh delivered after placement of the securement member rather than inserted together. It should be understood that the other embodiments disclosed herein would be inserted in a similar fashion. A delivery catheter 50 is inserted through an introducer sheath 100 in the femoral vein A and advanced through the septum to access the left atrial appendage B as shown in Figures 7 and 8. For insertion, the retention (securement) member 12 is in the collapsed position.

A pusher 51 is advanced distally from a proximal end of the catheter 50 to advance the device 10 from the catheter 50 as shown in Figures 8A and 8B. Alternatively, the catheter 50 is withdrawn (with the pusher abutting retention member 12) to expose the struts. As the struts 17 of the device 10 are exposed, they return toward their shape memorized deployed position to engage the appendage wall as shown in Figure 8B. The extent they return to their fully memorized position will depend on the size of the appendage.

In some embodiments, the retention member 12 will be positioned at the opening to the left atrial appendage B and be substantially flush with the opening. That is, the proximal retrieval hook would be positioned at the opening. Alternatively, a portion of the retention member 12 may extend proximally past the opening into the atrium as shown for example in Figure 8B. For example, as shown in Figure 8B, the device can have struts forming a wider base to conform to the shape of the appendage at the opening with the mesh floating up to the appendage opening. In this use, the portion of reduced transverse dimension remains outside the appendage. It is contemplated in some embodiments that the mesh or other clot material when expanded floats only in the large transverse dimension region of the retention member and is too large to float within the reduced dimension region, or a portion thereof. In such embodiments, the mesh or other clot material would thereby not extend outside the appendage, e.g. beyond the appendage opening, if placement of the retention member of Figure 8B is performed.

After placement within the appendage as shown in Figure 8B, the delivery catheter 50 is inserted through a space between the struts 17 and mesh 30 (or other clotting material ) is pushed out of the delivery catheter 50 for placement in the space between the struts 17 of device 10 for free floating movement therein (see Figure 8D). Delivery catheter 50 is then withdrawn. As can be appreciated, as an alternative to the clotting material retained in delivery catheter 50 proximal of the retention member 12 for delivery, after retention member 12 placement, the delivery catheter 50 can be withdrawn and another delivery device containing the clot material can be inserted and advanced to the left atrial appendage and through the struts 17 for delivery of the clotting material. Thus, for in situ delivery, the same catheter 50 or a different catheter can be utilized.

As can be appreciated, the material described in the embodiments herein preferably free floats within the struts of the retention member, causing the blood to clot which then prevents migration of thrombus from the appendage into the atrium and left ventricle. The clot material floating within the retention member is preferably thrombogenic.

Note the material inside the retention member could be made of various materials, including, but not limited to, pericardium, SIS, PET, PTFE, etc.

In the alternate embodiment of Figure 9, a wound wire 150 provides a retention (securement) member for mesh 160. The wire as shown has a substantially conical configuration so the diameter (transverse dimension) at region 152 exceeds the diameter (transverse dimension) of region 154. The floating mesh 160 is inside, preferably free floating. The wire could have hooks, barbs or other surfaces to enhance retention in addition to the outward radial force against the appendage. The ribbons, fibers, or other clotting materials as described above can be placed inside the wound wire and unattached thereto for floating movement to achieve the blood clot function in the some way as in the embodiments of Figures 1-6 described above. The clot material can be delivered with the wire 150, i.e. collapsed within the collapsed wire within the delivery sheath, or alternatively delivered with the same or different catheter after placement of the wire 150 in the appendage. Figure 9A illustrates placement of the wire 150 within the left trial appendage B.

As can be appreciated, although described for use in the left atrial appendage of the heart, the device can also be used in other conduits such as blood vessels, ureters of fistulas.

While the above description contains many specifics, those specifics should not be construed as limitations on the scope of the disclosure, but merely as exemplifications of preferred embodiments thereof. For example, other materials can be contained or within the retention member to function to cause blood clot to block clot migration from the left atrial appendage. Those skilled in the art will envision many other possible variations that are within the scope and spirit of the disclosure as defined by the claims appended hereto.

## Claims

1. A device for placement in the left atrial appendage (B) of a patient comprising a retention member (12, 112, 150, 212) having a first elongated configuration for delivery and a second expanded configuration for placement within the left atrial appendage, a material (30, 120, 160, 215) positioned within the retention member and unattached thereto for floating movement therein, in the expanded configuration of the retention member, the retention member having a larger transverse dimension, the retention member having at least one appendage wall engagement member (14, 14', 114, 214) to secure the retention member to the appendage.

2. The device of claim 1, wherein the retention member (12, 112, 150, 212) is composed of a shape memory material, and in the expanded configuration the retention member moves toward a shape memory position.

3. The device of claim 1 or 2, wherein the material comprises a mesh (30, 160).

4. The device of claim 1, 2 or 3, wherein the material comprises a plurality of intertwined fibers (120).

5. The device of any preceding claim, wherein the material comprises a plurality of intertwined ribbons (215).

6. The device of any preceding claim, wherein the retention member (12, 112, 212) has a plurality of struts (17, 117, 217) defining a space therebetween and the material (30, 120, 215) floats freely within the space.

7. The device of any preceding claim, wherein the engagement member (14, 114, 214) includes a plurality of teeth.

8. The device of any preceding claim, wherein the retention member (12, 112, 212) has a plurality of struts (17, 117, 217) and the struts terminate in the engagement members (14, 14', 114, 214).

9. The device of any preceding claim, wherein the retention member (150) comprises a wound wire.

10. A device for placement in the left atrial appendage (B), for example dependent on any preceding claim, wherein the device comprises a tube laser cut to form a series of struts (17, 117, 217), the tube having a first elongated configuration for delivery and a second expanded configuration for placement, the struts extending outwardly so that a distal region of the struts has a greater dimension than a proximal region, the struts defining a space therebetween, a material (30, 120, 160, 215) non attachably positioned within the space defined by the struts and floating therein for causing blood clots within the appendage.

11. The device of claim 10, wherein the material comprises a mesh (30, 160).

12. The device of claim 10 or 11, wherein the material comprises a plurality of intertwined fibers (120).

13. The device of claim 10, 11 or 12, wherein the material comprises a plurality of intertwined ribbons (215).

14. The device of any one of claims 10 to 13, wherein the struts (17, 117, 217) are composed of shape memory material.

15. The device of any one of claims 10 to 14, wherein the material (30, 120, 215) is positionable within the space defined by the struts (17, 117, 217) subsequent to placement of the struts in the appendage.
